# EUROPÄISCHE PATENTANMELDUNG

(11) **EP 3 404 108 A1**
(43) Veröffentlichungstag der Anmeldung: **21.11.2018**
(21) Anmeldenummer: 18179368.8
(22) Anmeldetag: 02.06.2010
(51) Int. Cl.: C12P 17/04, C12N 9/90

(54) **BIOKATALYTISCHE HERSTELLUNG VON AMBROXAN**

(30) Priorität: 05.06.2009 EP 09162104
(62) Teilanmeldung aus: 10724460.0
(71) Anmelder: BASF SE, 67056 Ludwigshafen am Rhein (DE)
(72) Erfinder: BREUER, Michael, 64285 Darmstadt (DE); HÖRSTER, Andrea, 67067 Ludwigshafen (DE); HAUER, Bernhard, 67136 Fußgönheim (DE)
(74) Vertreter: Fitzner, Uwe

(57) **Zusammenfassung**

Die Erfindung betrifft ein Verfahren zur biokatalytischen Herstellung von Ambroxan mittels eines Polypeptids mit der Aktivität einer Homofarnesol-Ambroxan-Cyclase, die eine neue Klasse von Enzymen darstellen.

## Beschreibung

Die Erfindung betrifft ein Verfahren zur biokatalytischen Herstellung von Ambroxan.

Verbindungen mit dem Grundgerüst des Dodecahydronaphtho[2,1-b]furans sind als Aromachemikalien von großem wirtschaftlichem Interesse. Hier ist Verbindung 2 zu nennen, das als das linksdrehende Stereoisomer [(-)-2] des Ambroxan bekannte (3a*R*,5a*S*,9a*S*,9b*R*)-3a,6,6,9a-Tetramethyldodecahydronaphtho-[2,1-*b*]-furan).

Ursprünglich aus dem Ambra des Pottwals gewonnen, gibt es heute vor allem zwei Routen, die Zugang zu Ambroxan erlauben. Sclareol (3), ein Inhaltsstoff des Muskatellersalbeis (*Salvia sclarea*) dient dabei oft als geeignetes Ausgangsmaterial für semisynthetisches Material, weil es die optische Information für Verbindung ((-)-2) bereits enthält. Dabei kann man den oxidativen Abbau mit Chromsäure, Permanganat, H2O2 oder Ozon durchführen [Stoll et al.; Helv Chim Acta (1950), 33: 1251]. Das erhaltene Sclareolid (4) wird durch eine anschließende Reduktion (z.B. mit LiAIH4 oder NaBH4) in das Ambrox-1,4-diol (5) überführt [Mookherjee et al.; Perfumer and F/avourist (1990), 15: 27]. Die Herstellung von Verbindung (4) aus Sclareol (3) kann auch durch eine Biotransformation mit *Hyphozyma roseoniger* erfolgen[EP 204009]. 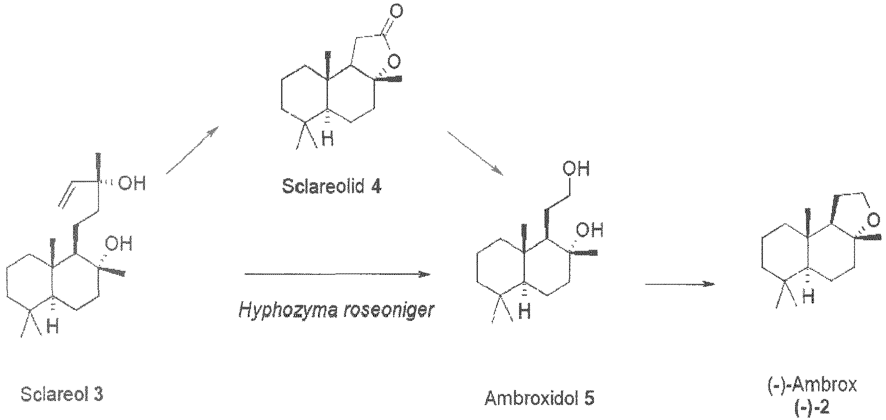 Ambrox-1,4-diol (5) kann schließlich mit einer Reihe von chemischen Verfahren zu Verbindung ((-)-2) cyclisiert werden. Zu dem Racemat von Ambroxan rac-2 sind Zugänge u.a. via Homofarnesylsäure [US 513,270; Lucius et al.; Chem Ber (1960), 93: 2663] und 4-(2,6,6-Trimethyl-cyclohex-1-enyl)-butan-2-on [Büchi et al.; Helv Chim Acta (1989), 72: 996] bearbeitet worden. Das Marktvolumen von Ambroxan lag 2002 zurzeit mittelwertig bei 20 Tonnen pro Jahr. Hierfür ist eine Ausgangsbasis von ca. 33 Tonnen Sclareol pro Jahr notwendig. Für die Produktion einer Tonne Ambroxan werden 207 Tonnen verschiedener Einzelstoffe benötigt, die wiederum den Anfall von 206 Tonnen Abfall bedingen. Die anfallenden Substanzen weisen unterschiedliche, insgesamt aber verhältnismäßig starke Umwelt- und Gesundheitswirkungen auf [Deutsche Bundesstiftung Umwelt]. Somit ist diese Synthese sehr energieaufwändig und erfordert den Einsatz umweltbelastender Chemikalien.

Die biokatalytische Synthese von Verbindung ((-)-2) ist in der Literatur beschrieben [Neumann et al.; Biol Chem Hoppe Seyler (1986), 367: 723]. Dabei wird das Molekül aus Homofarnesol (Verbindung (1), (3*Z*,7*E*)-4,8,12-Trimethyltrideca-3,7,11-trien-1-ol) gewonnen. Als Katalysator wurde die Squalen-Hopen-Cyclase (SHC) aus *Alicyclobacillus acidocaldarius* (ehem. *Bacillus acidocaldarius*) verwendet. Das Enzym katalysiert natürlicherweise die Zyklisierung von Squalen zu Hopan. Als Nebenreaktion kann diese SHC offenbar auch Verbindung (1) zu Ambroxan ((-)-2) umsetzen. Der Biokatalysator kann rekombinant hergestellt werden [Ochs D.et al.; J Bacteriol (1992), 174: 298]. Der Umsatz der Cyclisierung von Homofarnesol zu Ambroxan beträgt nach Neumann et al. allerdings lediglich 1,2% (berechnet aus den GC-peaks) und die spezifische Aktivität bezüglich der Cyclisierung von Homofarnesol wird mit 0,02mU/mg Protein angegeben.

Aufgabe der vorliegenden Erfindung war es somit ein neues Verfahren zur Herstellung von Ambroxan sowie Derivaten davon zur Verfügung zu stellen, das Vorteile gegenüber den technisch aufwendigen Synthesen des Standes der Technik bietet. Durch den Einsatz biotechnischer Verfahren sollte das Entstehen von Umweltgiften vermieden und der Energieaufwand drastisch reduziert werden. Weitere Aufgabe war es die anfallenden Kosten zusätzlich durch die Verwendung leicht zugänglicher Ausgangsstoffe sowie einer Verminderung der Anzahl chemischer Reaktionen (bzw. Stufen) zu senken. Ferner sollte eine erhöhte Produktivität erreicht werden.

Diese Aufgabe wird gelöst durch ein Verfahren zur Herstellung von Ambroxan-Derivaten, bevorzugt Ambroxan, der allgemeinen Formel (2) dadurch gekennzeichnet, dass Homofarnesol-Derivate der allgemeinen Formel (1) biokatalytisch zu den entsprechenden Ambroxan-Derivaten mittels eines Polypeptids mit der Aktivität einer Homofarnesol-Ambroxan-Cyclase als Enzym umgesetzt werden.

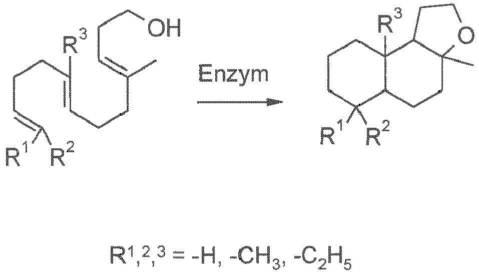

Derivate sind insbesondere Stereoisomere, bevorzugt Enantiomere aber auch Diastereomere der Verbindung (2). In einer Ausführung der vorliegenden Erfindung sind Derivate von Homofarnesol bzw. Ambroxan substituierte Verbindungen (1) und (2), wobei die Substituenten bezüglich der biokatalysierten Umsetzung inert sind. Damit sind insbesondere Verbindungen der unten dargestellten Strukturformeln gemeint.

Im Sinne der Erfindung werden die Begriffe "Verbindung (1), "Homofarnesol", "4,8,12-Trimethyl-trideca-3,7,11-trien-1-ol)" und Derivate des Homofarnesols bzw. die Begriffe "Verbindung (2)", "Ambroxan", "Dodecahydronaphtho[2,1-b]furan" und Derivate des Ambroxans gleichbedeutend und gegeneinander austauschbar und ersetzbar, sofern nichts Gegenteiliges ausdrücklich definiert wird.

In einer bevorzugten Variante der Erfindung wird das linksdrehende Ambroxan der Formel ((-)-2) gebildet:

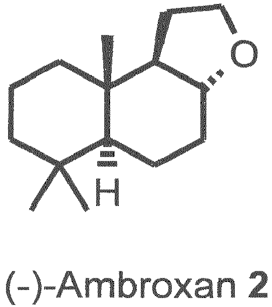

Polypeptide mit der Aktivität einer Homofarnesol-Ambroxan-Cyclase stellen eine neue Klasse von Enzymen dar.

Der Begriff "Aktivität" beschreibt die Fähigkeit eines Enzyms, ein Substrat in ein Produkt umzuwandeln. Die Aktivität kann in einem sogenannten Aktivitätstest über die Zunahme des Produktes, die Abnahme des Substrates (oder Eduktes) oder über eine Kombination dieser Parameter in Abhängigkeit von der Zeit bestimmt werden.

Die erfindungsgemäßen Enzyme sind dadurch charakterisiert, dass ihre Aktivität die Umsetzung von Homofarnesol zu Ambroxan ist.

Im Sinne der Erfindung ist in einer Variante das Hauptsubstrat die chemische Verbindung, welche im Vergleich zu allen anderen Verbindungen, die von dem Enzym umgesetzt werden können, in Molprozent den größten Anteil als Reaktionspartner der Homofarnesol-Ambroxan-Cyclase aufweist.

Im Sinne der Erfindung ist in einer Variante das Hauptsubstrat Homofarnesol und somit die Hauptaktivität und damit die Hauptreaktion einer Homofarnesol-Ambroxan-Cyclase die Reaktion mit dem Hauptsubstrat Homofarnesol.

Die Aktivität der Homofarnesol-Ambroxan-Cyclase wird in einer Variante der Erfindung über die Ausbeute in Molprozent definiert. Bevorzugt erhält man bei der Umsetzung von Homofarnesol zu Ambroxan in Gegenwart einer Enzyms der neuen Klasse der Homofarnesol-Ambroxan-Cyclasen eine Ausbeute an Ambroxan in Molprozent bezogen auf die eingesetzten Mol Homofarnesol von 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100; besonders bevorzugt liegt die Ausbeute zwischen 5 und 100, 10 und 100, 20 und 100, 25 und 100, 30 und 100, 35 und 100, insbesondere zwischen 40 und 100, 45 und 100, 50 und 100, 60 und 100, 70 und 100 Molprozent.

Die Aktivität der Homofarnesol-Ambroxan-Cyclase wird in einer weiteren Variante der Erfindung über den Umsatz (Produktmenge/(Produktmenge + Resteduktmenge) * 100) in Molprozent definiert. Bevorzugt erhält man bei der Umsetzung von Homofarnesol zu Ambroxan in Gegenwart einer Enzyms der neuen Klasse der Homofarnesol-Ambroxan-Cyclasen einen Umsatz an Ambroxan in Molprozent von 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100; besonders bevorzugt liegt der Umsatz zwischen 5 und 100, 10 und 100, 20 und 100, 25 und 100, 30 und 100, 35 und 100, insbesondere zwischen 40 und 100, 45 und 100, 50 und 100, 60 und 100, 70 und 100 .

In einer bevorzugten Ausführung der Erfindung wird die Ausbeute und/oder der Umsatz über einen definierten Zeitraum von zum Beispiel 4, 6, 8, 10, 12, 16, 20, 24, 36 oder 48 Stunden bestimmt in dem die Umsetzung von Homofarnesol zu Ambroxan mittels der erfindungsgemäßen Cyclasen erfolgt. In einer weiteren Variante wird die Umsetzung unter genau definierten Bedingungen durchgeführt, von zum Beispiel 25, 30, 40, 50 oder 60°C. Insbesondere erfolgt die Bestimmung der Ausbeute und/oder des Umsatzes indem die Reaktion zur Umsetzung von Homofarnesol zu Ambroxan mittels der erfindungsgemäßen Cyclasen bei 30°C über 16 Stunden durchgeführt wird.
In einer Ausführung der Erfindung werden zur Bestimmung der Ausbeute und/oder des Umsatzes eine 10 mM Homofarnesol-Lösung (Citrat-gepuffert) mit einer Cyclase-Lösung umgesetzt wird, wobei das Enzym als Membran-Proteinextrakt Homofarnesol-Ambroxan-Cyclasen exprimierender Zellen (isoliert gemäß [Ochs D.et al.; J Bacteriol (1992), 174: 298]) in einer Konzentration an Proteingehalt von 0,08 Gewichtsprozent vorliegt.
In einer weiteren Ausführung der vorliegenden Erfindung wird eine Homofarnesol-Ambroxan-Cyclase dadurch gekennzeichnet, daß sie bei der Umsetzung von Homofarnesol zu Ambroxan unter den gleichen Bedingungen die 2-, 3-, 4-, 5-, 6-, 7-, 8-, 9-, 10-, 11-, 12-, 13-, 14-, 15-, 16-, 17-, 18-, 19-, 20-, 21-, 22-, 23-, 24-, 25-, 26-, 27-, 28-, 29-, 30-, 31-, 32-, 33-, 34-, 35-, 36-, 37-, 38-, 39-, 40-, 41-, 42-, 43-, 44-, 45-, 46-, 47-, 48-, 49-, 50-, 51-, 52-, 53-, 54-, 55-, 56-, 57-, 58-, 59-, 60-, 61-, 62-, 63-, 64-, 65-, 66-, 67-, 68-, 69-, 70-, 71-, 72-, 73-, 74-, 75-, 76-, 77-, 78-, 79-, 80-, 81-, 82-, 83-, 84-, 85-, 86-, 87-, 88-, 89-, 90-, 91-, 92-, 93-, 94-, 95-, 96-, 97-, 98-, 99-, 100-, 200-, 500-, 1000-fache oder höhere Ausbeute und/oder des Umsatz im Vergleich zu der Squalen-Hopen-Cyclase (SHC) aus *Alicyclobacillus acidocaldarius* (ehem. *Bacillus acidocaldarius*) zeigt. Der Begriff Bedingung bezieht sich hier auf Reaktionsbedingungen wie Susbstratkonzentration, Enzamkonzentration, Umsetzungszeitraum und/oder Temperatur.

In einer Variante der vorliegenden Erfindung wird eine Homofarnesol-Ambroxan-Cyclase durch jede oder mehrere, in jeder beliebigen Kombination, der oben genannten Definitionen charakterisiert.

Die Erfindung betrifft auch ein Verfahren zur Herstellung von Ambroxan, bei dem
a) Homofarnesol mit der Homofarnesol-Ambroxan-Cyclase in Kontakt gebracht und/oder inkubiert wird, und
b) Ambroxan isoliert wird.

In einer Ausführung der Erfindung wird Homofarnesol mit der Homofarnesol-Ambroxan-Cyclase in einem Medium in Kontakt gebracht und/oder so inkubiert, dass eine Umsetzung von Homofarnesol zu Ambroxan in Gegenwart der Cyclase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von vorzugsweise von 5 bis 8, aufweisen. Als Puffer kann ein Citrat-, Phosphat-, TRIS (Tris(hydroxymethyl)-aminomethan), MES (2-(N-Morpholino)ethansulfonsäure), Puffer verwendet werden. Ferner kann das Reaktionsmedium noch weitere Zusätze enthalten, wie z.B. Detergentien (beispielsweise Taurodeoxycholat).

Das Substrat (1) wird vorzugsweise in einer Konzentration von 5 - 100mM, besonders bevorzugt von 15 - 25mM in die enzymatische Umsetzung eingesetzt und kann kontinuierlich oder diskontinuierlich nachgeführt werden.

Die enzymatische Cyclisierung erfolgt in der Regel bei einer Reaktionstemperatur unterhalb der Desaktivierungstemperatur der eingesetzten Cyclase und oberhalb von -10 °C. Besonders bevorzugt liegt sie im Bereich von 0 bis 100 °C, insbesondere von 15 bis 60 °C und speziell von 20 bis 40 °C, z.B. bei etwa 30 °C.

Das Reaktionsprodukt Ambroxan kann mit organischen Lösungsmitteln, ausgewählt aus der Gruppe der unten genannten, extrahiert werden und gegebenenfalls zur Aufreinigung destilliert werden.

Neben diesen einphasigen wässrigen Systemen werden in einer weiteren Variante der Erfindung auch zweiphasige Systeme eingesetzt. Dabei werden ionische Flüssigkeiten als zweite Phase, bevorzugt aber organische, nicht-wasser-mischbare Reaktionsmedien als zweite Phase verwendet. Dadurch akkumulieren die Reaktionsprodukte in der organischen Phase. Nach der Reaktion ist Ambroxan in der organische Phase leicht von der wässrigen Phase, die den Biokatalysator enthält, abtrennbar.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

In einer Ausführung der vorliegenden Erfindung wird als Ausgangsstoff für die Ambroxan-Synthese Citral eingesetzt. Dies ist ein besonderer Vorteil des erfindungsgemäßen Verfahrens, da Citral in großen Mengen kostengünstig verfügbar ist. Citral wird in einer klassischen chemischen Synthese zu Homofarnesol umgesetzt

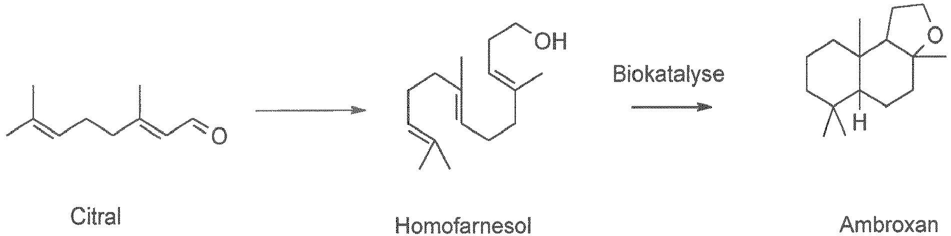

In einer Ausführung der vorliegenden Erfindung erfolgt die Umsetzung . Citral zu Homofarnesol umgesetzt in folgenden Schritten .(z.B.: JOC, (1992), 57, 2794):
Das erfindungsgemäße Verfahren bietet dadurch einen weiteren Vorteil, dass die gesamte Umsetzung von Homofarnesol zu Ambroxan in einphasigen wässrigen Systemen aber auch in 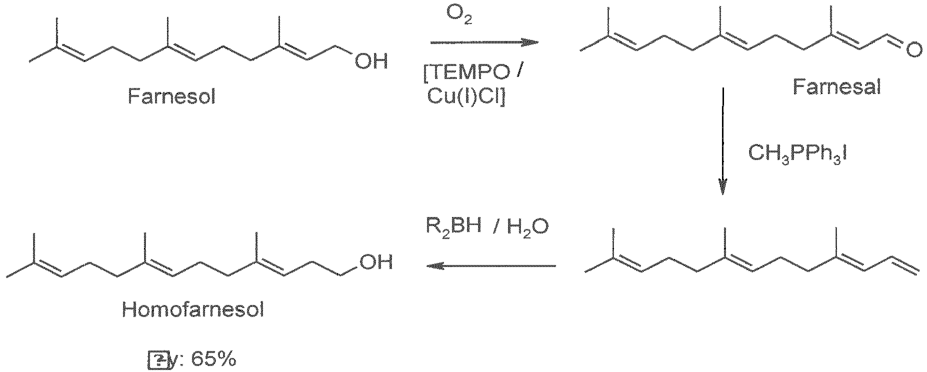 zweiphasigen Systemen stattfindet. Bei den zweiphasigen Systemen werden die oben genannten eingesetzt. Bevorzugt werden die oben genannten organische, nicht-wasser-mischbare Lösungsmittel als zweite Phase verwendet. Dadurch akkumulieren die Reaktionsprodukte in der organischen Phase. Nach der Reaktion ist Ambroxan in der organische Phase leicht von der wässrigen Phase, die den Biokatalysator enthält, abtrennbar.

Auch in der Ausführung der Erfindung mit Citral als Ausgangsstoff, wird Homofarnesol mit der Homofarnesol-Ambroxan-Cyclase in einem Medium in Kontakt gebracht und/oder so inkubiert, dass eine Umsetzung von Homofarnesol zu Ambroxan in Gegenwart der Cyclase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von zugsweise von 5 bis 8, aufweisen. Als Puffer kann ein Citrat-, Phosphat-, TRIS (Tris(hydroxymethyl)-aminomethan), MES (2-(N-Morpholino)ethansulfonsäure) Puffer verwendet werden. Ferner kann das Reaktionsmedium noch weitere Zusätze enthalten, wie z.B. Detergentien (Taurodeoxycholat o.ä.).
In einer Ausführung der Erfindung wird das Reaktionsprodukt Ambroxan mit organischen Lösungsmitteln, ausgewählt aus der Gruppe der unten genannten, extrahiert werden und gegebenenfalls zur Aufreinigung destilliert werden.

Ein weiterer Gegenstand der vorliegenden Erfindung ist ein Verfahren zur biokatalytischen Herstellung von Ambroxan, dadurch gekennzeichnet, dass das Enzym ein Polypeptid ist, welches kodiert wird von einem Nukleinsäuremolekül umfassend mindestens ein Nukleinsäuremolekül ausgewählt aus der Gruppe bestehend aus:
a) Nukleinsäuremolekül, das ein Polypeptid kodiert, umfassend die in SEQ ID NO 2, 5, 6, 7, 8, 9, 10 oder 11;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Bank oder aus genomischer DNA mittels der Primer gemäß Sequenz Nr. 3 und 4 amplifiziert, oder das Nukleinsäuremolekül durch *de novo* Synthese chemisch synthetisiert;
f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

Eine analoge oder ähnliche Bindungsstelle im Sinne der Erfindung ist eine konservierte Domäne oder Motiv der Aminosäurensequenz mit einer Homologie von 80%, besonders bevorzugt 85%, 86%, 87%, 88%, 89%, 90%, insbesondere 91%, 92%, 93%, 94%, 95%, 96%, 97%, 98% oder 99% oder 100%, welche die Bindung desselben Substrats, insbesondere Homofarnesol gewährleistet.

Bevorzugt weist das Nukleinsäuremolekül c) eine Identität mit der SEQ ID NO:1 von mindestens 46%, 47%, 48%, 49%, 50%, 55%, 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% auf.

Ebenso weisen ein funktional äquivalentes Polypeptid eine Identität mit der SEQ ID NO: 2 von mindestens 46%, 47%, 48%, 49%, 50%, 55%" 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% auf.

Anstelle des Begriffs "Identität" kann auch der Begriff "homolog" oder "Homologie" gleichbedeutend verwendet werden.

Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet.

Bevorzugt wird die Identität zwischen zwei Nukleinsäuresequenzen oder Polypeptidsequenzen durch die Identität der Nukleinsäuresequenz/Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453)) berechnet wird.
Bevorzugt werden die Identitätsvergleiche unter Einstellung folgender Parameter für Aminosäuren:
Gap creation penalty: 8; Gap extension penalty: 2
und die folgenden Parameter für Nukleinsäuren:
   Gap creation penalty: 50; Gap extension penalty: 3 durchgeführt.
In einer Ausführung der Erfindung wird die Identität zwischen zwei Nukleinsäuresequenzen oder Polypeptidsequenzen durch Vergleich mit Hilfe des Programms BLASTP 2.2.20+ mit Standard-Einstellungen wie unter NCBI Blast vorgeschlagen (Reference: Altschul et al., (1997), Nucleic Acids Res. 25:3389-3402. Reference for compositional score matrix adjustment: Altschul et al., (2005), FEBS J. 272:5101-5109.)

Gegenstand der Erfindung sind weitere Homologe oder funktionelle Äquivalente der SEQ ID NO: 1, die mit dieser Nukleinsäuresequenz unter stringenten Bedingungen hybridisieren.

"Funktionelle Äquivalente" beschreiben prinzipiell hier Nukleinsäuresequenzen, die unter Standardbedingungen mit einer Nukleinsäuresequenz oder Teilen einer Nukleinsäuresequenz hybridisieren und befähigt sind, die Expression eines Proteins mit den gleichen Eigenschaften wie die der Homofarnesol-Ambroxan-Cyclase in einer Zelle oder einem Organismus zu bewirken.

Zur Hybridisierung werden vorteilhaft kurze Oligonukleotide mit einer Länge von etwa 10-50 bp, vorzugsweise 15-40 bp beispielsweise der konservierten oder sonstigen Bereiche, die über Vergleiche mit anderen verwandten Genen in dem Fachmann bekannter Weise ermittelt werden können, verwendet. Es können aber auch längere Fragmente der erfindungsgemäßen Nukleinsäuren mit einer Länge von 100-500 bp oder die vollständigen Sequenzen für die Hybridisierung verwendet werden. Je nach der verwendeten Nukleinsäure/Oligonukleotid, der Länge des Fragmentes oder der vollständigen Sequenz oder je nachdem welche Nukleinsäureart, d.h. DNA oder RNA, für die Hybridisierung verwendet werden, variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardhybridisierungsbbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20°C bis 65°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 65°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50% in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, "Current Protocols in Molecular Biology", John Wiley & Sons, New York; Harnes and Higgins (eds), 1985, "Nucleic Acids Hybridization: A Practical Approach", IRL Press at Oxford University Press, Oxford; Brown (ed), 1991, Essential Molecular Biology: A Practical Approach, IRL Press at Oxford University Press, Oxford.

Unter einem funktionellen Äquivalent versteht man weiterhin auch Nukleinsäuresequenzen, die mit einer bestimmten Nukleinsäuresequenz ("ursprüngliche Nukleinsäuresequenz) bis zu einem definierten Prozentsatz homolog bzw. identisch sind und die gleiche Aktivität wie die ursprünglichen Nukleinsäuresequenzen aufweisen, ferner insbesondere auch natürliche oder künstliche Mutationen dieser Nukleinsäuresequenzen.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Hauptaktivität, Substratspezifität, besitzen. So versteht man beispielsweise unter "funktionalen Äquivalenten" Enzyme, die die Modellreaktion katalysieren und die mindestens 20 %, 30%, 50%, 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% der Aktivität eines Enzyms, umfassend eine unter SEQ ID NO: 2, 5, 6, 7, 8, 9, 10 oder 11 aufgeführten Aminosäuresequenzen, aufweist.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

| | |
|---|---|
| Ile; Leu"Funktionale Äquivalente" im obigen Sinne sind auch "Präkursoren" der beschriebenen Polypeptide sowie "funktionale Derivate" . | |

"Präkursoren" sind dabei natürliche oder synthetische Vorstufen der Polypeptide mit oder ohne der gewünschten biologischen Aktiviät.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.
"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.
"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Gegenstand der Erfindung sind weiterhin Nukleinsäuresequenzen (einzel- und doppelsträngige DNA- und RNA-Sequenzen, wie z.B. cDNA und mRNA), die für ein Enzym mit erfindungsgemäßer Cyclase -Aktivität kodieren. Bevorzugt sind Nukleinsäuresequenzen, welche z.B. für Aminosäuresequenzen gemäß SEQ ID NO: 2, 5, 6, 7, 8, 9, 10 oder 11 charakteristische Teilsequenzen davon kodieren.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Weitere Ausgestaltungen zur Durchführung des erfindungsgemäßen biokatalytischen Verfahrens zur Herstellung von Ambroxan:
Das erfindungsgemäße Verfahren ist dadurch gekennzeichnet, dass das Enzym in einer Form vorliegt, ausgewählt aus der Gruppe bestehend aus:
a) freies, gegebenenfalls gereinigtes oder teilweise gereinigtes Polypeptid mit der Aktivität einer Homofarnesol-Ambroxan-Cyclase;
b) immobilisiertes Polypeptid mit der Aktivität einer Homofarnesol-Ambroxan-Cyclase;
c) aus Zellen isoliertes Polypeptid gemäß a) oder b);
d) ganze Zelle, gegebenenfalls ruhende oder aufgeschlossene Zellen, enthaltend mindestens ein Polypeptid mit der Aktivität einer Homofarnesol-Ambroxan-Cyclase;
e) Zelllysat oder Zellhomogenisat der Zellen beschrieben unter d).

In einer Ausführung der Erfindung sind die Zellen Mikroorganismen, bevorzugt transgene Mikroorganismen exprimierend mindestens ein heterologes Nukleinsäuremolekül kodierend für ein Polypeptid mit der Aktivität einer Homofarnesol-Ambroxan-Cyclase.

Weiterer Gegenstand der vorliegenden Erfindung sind somit auch ein Genkonstrukt oder ein Vektor umfassend ein Nukleinsäuremolekül kodierend für ein Polypeptid mit der Aktivität einer Homofarnesol-Ambroxan-Cyclase, bevorzugt ein Nukleinsäuremolekül umfassend mindestens ein Nukleinsäuremolekül ausgewählt aus der Gruppe bestehend aus:
a) Nukleinsäuremolekül, das ein Polypeptid kodiert, umfassend die in SEQ ID NO 2, 5, 6, 7, 8, 9, 10 oder 11;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
d) Nukleinsäuremolekül nach (a) bis (c), das für ein Fragment der Sequenzen gemäß SEQ ID NO 2, 5, 6, 7, 8, 9, 10 oder 11;
e) Nukleinsäuremolekül, kodierend für ein Polypeptids mit der Aktivität einer Homofarnesol-Ambroxan-Cyclase, das man erhält in dem man einen Nukleinsäuremolekül aus einer cDNA Datenbank oder aus einer Genom-Datenbank mittels der Primer gemäß Sequenz Nr. 3 und 4 amplifiziert;
f) Nukleinsäuremolekül, kodierend für ein Polypeptids mit der Aktivität einer Homofarnesol-Ambroxan-Cyclase, das unter stringenten Bedingungen mit einem Nukleinsäuremolekül gemäß (a) bis (c) hybridisiert;
g) Nukleinsäuremolekül kodierend für ein Polypeptids mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein Polypeptids mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

Ferner sind die Wirtszellen enthaltend ein Genkonstrukt oder einen Vektor wie oben beschrieben ebenfalls Gegenstand der vorliegenden Erfindung. Hierzu werden die verwendeten Nukleinsäuresequenzen vorteilhaft in ein transgenes Genkostrukt eingebracht, die eine transgene Expression einer Homofarnesol-Ambroxan-Cyclase, in einem Organismus, bevorzugt Mikroorganismus gewährleisten können.

In den Genkostrukten steht dabei ein Nukleinsäuremolekül kodierend für eine Homofarnesol-Ambroxan-Cyclasebevorzugt in funktioneller Verknüpfung mit mindestens einem genetischen Kontrollelement (beispielsweise einem Promotor und/oder Terminator), das eine Expression in einem Organismus, bevorzugt Mikroorganismus gewährleistet.

Unter einer funktionellen Verknüpfung versteht man zum Beispiel die sequentielle Anordnung eines Promotors mit der zu exprimierenden Nukleinsäuresequenz kodierend für eine Homofarnesol-Ambroxan-Cyclase (zum Beispiel der Sequenz gemäß SEQ ID NO: 1) und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Die Herstellung einer funktionellen Verknüpfung als auch die Herstellung des Genkonstruktes kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann das Genkonstrukt, bestehend aus einer Verknüpfung von Promoter und zu exprimierender Nukleinsäuresequenz, integriert in einem Vektor vorliegen und durch zum Beispiel Transformation in einen Mikroorganismus insertiert werden.

Die in den Genkonstrukten oder Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluss auf das Zustandekommen oder die Funktion des Genkonstruktes haben. Genetische Kontrollsequenzen modifizieren zum Beispiel die Transkription und Translation in prokaryotischen oder eukaryotischen Organismen. Vorzugsweise umfassen die Genkonstrukte eine Kontrollsequenz, sowie gegebenenfalls weitere übliche regulative Elemente, und zwar jeweils funktionell verknüpft mit der transgen zu exprimierenden Nukleinsäuresequenz.

Als Kontrollsequenzen sind solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel die kodierende Sequenz eines bestimmten endogenen Gens gegen die für eine dsRNA kodierende Sequenz gezielt ausgetauscht werden.

Ein Genkonstrukt und die von ihr abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten, Vektoren oder transgenen Organismen haben. Beispielhaft aber nicht einschränkend seien zu nennen:
a) Selektionsmarker, die eine Resistenz gegen Antibiotika oder Biozide wie zum Beispiel Kanamycin, G 418, Bleomycin, Hygromycin, etc. verleihen.
b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al.(1995) Plant Journal 8(5):777-784), die Chloramphenicoltransferase, eine Luziferase (Ow et al. (1986) Science 234:856-859), das Aequorin-Gen (Prasher et al. (1985) Biochem Biophys Res Commun 126(3):1259-1268), die beta-Galactosidase, ganz besonders bevorzugt ist die ß-Glucuronidase (Jefferson et al. (1987) EMBO J 6:3901-3907).
c) Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid oder ein Antibiotikum verleiht. Der Selektionsmarker erlaubt die Selektion der transformierten Zellen von untransformierten (McCormick et al. (1986) Plant Cell Reports 5:81-84).

Die Einführung des Genkonstruktes in einen Organismus kann vorteilhaft unter Verwendung von Vektoren realisiert werden, in denen die Genkonstrukte enthalten sind. Ein weiterer Gegenstand der Erfindung betrifft daher besagte transgene Vektoren, die ein transgens Genkonstrukt für eine Homofarnesol-Ambroxan-Cyclase umfassen.

Vektoren können beispielhaft Plasmide, Cosmide, Phagen, Viren sein. Das Genkonstrukt kann in den Vektor (bevorzugt ein Plasmidvektor) über eine geeignete Restriktionsschnittstelle eingeführt werden. Der entstandene Vektor wird zunächst in E.coli eingeführt. Korrekt transformierte E.coli werden selektioniert, gezüchtet und der rekombinante Vektor mit dem Fachmann geläufigen Methoden gewonnen. Restriktionsanalyse und Sequenzierung können dazu dienen, den Klonierungsschritt zu prüfen. Bevorzugt sind solche Vektoren, die eine stabile Integration der Expressionskassette in das Wirtsgenom ermöglichen.

Die Herstellung eines entsprechenden transgenen Organismus erfolgt z.B. mittels Transformation oder Transfektion mittels der entsprechenden Proteine oder Nukleinsäuren. Die Herstellung eines transformierten Organismus (bzw. einer transformierten Zelle) erfordert, dass die entsprechende DNA (z.B. der Expressionsvektor), RNA oder Protein in die entsprechende Wirtszelle eingebracht wird. Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika (wie Kanamycin, G 418, Bleomycin, Hygromycin etc.) zu verleihen vermag (s.o.). Transformierte Zellen, die ein solches Markergen exprimieren, sind in der Lage, in der Gegenwart von Konzentrationen eines entsprechenden Antibiotikums zu überleben, die einen untransformierten Wildtyp abtöten. 89:525-533 verwendet.

"Transgen" oder "recombinant" meint bezüglich zum Beispiel einer Nukleinsäuresequenz, eines Genkonstruktes oder einem Vektor enthaltend besagte Nukleinsäuresequenz oder einem Organismus transformiert mit besagter Nukleinsäuresequenz, Genkonstrukt oder Vektor alle solche durch gentechnische Methoden zustandegekommene Konstruktionen, in denen sich entweder
a) die Nukleinsäuresequenz kodierend für eine Homofarnesol-Ambroxan-Cyclase , oder
b) eine mit besagter Nukleinsäuresequenz unter a) funktionell verknüpfte genetische Kontrollsequenz, zum Beispiel ein funktioneller Promotor, oder
c) (a) und (b)
sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten.

Als transgene Organismen bevorzugte Wirts- oder Ausgangsorganismen sind vor allem Mikroorganismen gemäß der oben genannten Definition. Eingeschlossen sind im Rahmen der Erfindung insbesondere Mikroorganismen, bevorzugt solche transgenen oder rekombinanten Zellen, die aus Bakterien, Cyanobakterien, Pilzen und Hefen ausgewählt sind. Bevorzugt ist die Zelle ausgewählt aus Bakterien der Gattungen Escherichia, Corynebacterium, Ralstonia, Clostridium, Pseudomonas, Bacillus, Zymomonas, Rhodobacter, Streptomyces, Burkholderia, Lactobacillus und Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor und Zymomonas mobilis.

Als Spenderorganismus, d.h. Organismus aus dem eine Homofarnesol-Ambroxan-Cyclase isoliert wird, werden Methylococcus capsalatus, Rhodopseudomonas palustris, Bradyrhizobium japonicum, Frankia spec., Streptomyces coelicolor, Rhodopseudomonas palustris, Rhodopseudomonas palent, Frankia alni, Bacillus anthracis, Burkholderia ambifaria, insbesondere Zymomonas mobilis und Bradyrhizobium japonicum.

Die transgenen Organismen, insbesondere Streptomyces coelicolor oder Zymomonas mobilis, die endogene Homofarnesol-Ambroxan-Cyclasen aufweisen, zeigen in einer Variante der Erfindung eine Überexpression der Homofarnesol-Ambroxan-Cyclasen.
Überexpression bedeutet jede Form der Expression der Homofarnesol-Ambroxan-Cyclasen die zusätzlich zu der ursprünglichen Expression des Wildtyps zu finden ist.

In einer Ausführung der Erfindung betrifft die Herstellung transgener E. coli, die das Gen kodierend für die Homofarnesol-Ambroxan-Cyclasen exprimieren, bevorzugt SEQ ID NO 1. Hierzu wird mit Primern, z.B. Zm-SHC_fw und Zm-SHC_rev (SEQ ID NO 3 bzw. 3) das Gen der Cyclase, bevorzugt aus *Zymomonas mobilis* (SEQ ID NO 1) amplifiziert. Die Primer werden equimolar gemischt. Die PCR mit genomischer DNA eines Spendeorganismus, bevorzugt aus *Z. mobilis*(LU8910 = ATCC31821) werden nach Herstellerangaben mit Pwo-Polymerase (Roche Applied Science) und dem folgenden Temperatur-Gradienten-Programm durchgeführt: 95°C für 3 min; 30 Zyklen mit 95°C für 30 sec, 50°C für 30 sec, und 72°C für 3 min; 72°C für 10 min.; 4°C bis zur Verwendung. Das PCR-Produkt wird durch Agarosegel-Elektrophorese (1,2%E-Gel, Invitrogen) und Säulen-Chromatographie (GFX-Kit, Amersham Pharmacia) isoliert und anschließend sequenziert (Sequenzierprimer: Zm-SHC_fw und Zm-SHC_rev). Das PCR-Produkt wurde mit den Restriktionsendonukleasen, bevorzugt *Nde*I und *Bam*HI*,* verdaut und in entsprechend geschnittenen Vektor, bevorzugt *p*DHE1650-Vekto [WO 200132890 A1] ligiert wird. Das so erhaltene Plasmid wird in E. coli, bevorzugt in den Stamm *E. coli*TG10 *p*Agro4 [Takeshita S.et al.; Gene (1987), 61: 63] *p*HSG575 transformiert.

Aus einer entsprechenden Vorkultur angeimpft, werden die *E*. *coli* kultiviert, bevorzugt in LBAmp/Spec/Cm (100µg/l Ampicillin; 100µg/l Spectinomycin; 20µg/l Chloramphenicol), 0,1 mM IPTG, 0,5g/L Rhamnose in 16 h bei 37°C angezogen, und anschließend bei 5000*g/10min zentrifugiert und gegebenenfalls bei 4°C gelagert.

In einer weiteren Ausführung der Erfindung wird die Cyclase aus dem Spendeorganismus oder aus dem transgenen Wirtsorganismus isoliert und gegebenenfalls gereinigt.

Aus den Zellen wird ein Proteinextrakt hergestellt indem das Zellpellet in Aufschlusspuffer (0,2M Tris/HCl, 0,5M EDTA, pH 8.0), 375U Benzonase (z.B. Novagen, 25U/µL), 40µL PMSF (100mM, gelöst in *i*-PropOH), 5,3g/100 ml Saccharose und ca. 3,3mg/100 ml Lysozym suspendiert wird. Den Ansatz wird gemischt und 30min auf Eis inkubiert. Anschließend wird gegebenenfalls die Mischung bei -20°C eingefroren werden. Nach dem Auftauen des Ansatzes wird mit Aqua dest. aufgefüllt und nochmals für 30min auf Eis inkubiert. Anschließend werden die Zellen 3mal 3min mit Ultraschall aufgeschlossen.

Nach dem Aufschluss wurden die Zelltrümmer in 60min bei 4°C und 26900 **g* abzentrifugiert. Der Überstand wird verworfen und das Pellet in Solubilisationspuffer (50mM Tris/HCl, 10mM MgCl2x6H2O, 1% Triton X-100, pH 8.0) resuspendiert und ca. 5min homogenisiert, z.B. mit einem Potter. Anschließend wird die Suspension für 30min auf Eis gehalten. Der homogenisierte Extrakt wird erneut für 1h bei 4°C und 26900 * *g* zentrifugiert und das Pellet verworfen. Der Extrakt kann für Enzymtests eingesetzt werden und kann mehrere Wochen ohne Aktivitätsverlust bei -20°C gelagert werden. Der Proteingehalt liegt im Bereich von 1 mg /mL

Die erfindungsgemäß eingesetzten Cyclasen können im erfindungsgemäßen Verfahren als freies oder immobilisiertes Enzym verwendet werden.

Die erfindungsgemäß verwendeten Cyclasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Cyclase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Es können weiterhin ganze Zelle, gegebenenfalls ruhende oder aufgeschlossene Zellen, Zelllysat oder Zellhomogenisat verwendet werden.

Für das erfindungsgemäße Verfahren können wachsende Zellen verwendet werden, die für die Cyclase kodierenden Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Reaktion kann sowohl in *batch-* als auch in *fed-batch*-Fahrweise durchgeführt werden.

Das Produkt wird anschließend durch Extraktion und/oder Destillation gereinigt.

Weiterer Gegenstand der vorliegenden Erfindung ist die Verwendung eines Polypeptids mit der Aktivität einer Homofarnesol-Ambroxan-Cyclase zur biokatalytischen Umsetzung von Homofarnesol zu Ambroxan.
Außerdem ist Gegenstand der vorliegenden Erfindung die Verwendung eines Polypeptids mit der Aktivität einer Homofarnesol-Ambroxan-Cyclase zur biokatalytischen Umsetzung von Homofarnesol zu Ambroxan, dadurch gekennzeichnet, dass das Polypeptids kodiert wird von einem Nukleinsäuremolekül umfassend mindestens ein Nukleinsäuremolekül ausgewählt aus der Gruppe bestehend aus:
a) Nukleinsäuremolekül, das ein Polypeptid kodiert, umfassend die in SEQ ID NO 2, 5, 6, 7, 8, 9, 10 oder 11;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Datenbank oder aus einer Genom-Datenbank mittels der Primer gemäß Sequenz Nr. 3 und 4 amplifiziert oder das entsprechende Gen synthetisch herstellt;
f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.
Die nachfolgenden Beispiele sollen die Erfindung veranschaulichen, ohne sie jedoch einzuschränken. Hierbei wird auf beiliegende Abbildungen Bezug genommen, dabei zeigt:

### Experimenteller Teil

### Beispiele

### Beispiel 1 Klonierung der Zm-SHC und Expression in E. coli

Mit den Oligonukleotiden Zm-SHC_fw und Zm-SHC_rev kann das Gen der Cyclase aus *Zymomonas mobilis* amplifiziert werden.

### Primer:

| Primer Nr. | Sequenz (5'->3') | Position |
|---|---|---|
| Zm-SHC_fw | gcgctgtttcatatgggtattgaca | N-Term-Primer |
| Zm-SHC_rev | gcgcttaccctggatcctcgaaaat | C-Term-Primer |

Je 100 ng der Primer Zm-SHC_fw und Zm-SHC_rev wurden equimolar gemischt. Die PCR mit genomischer DNA aus *Z. mobilis* (ATCC31821) wurde nach Herstellerangaben mit Pwo-Polymerase (Roche Applied Science) und dem folgenden Temperatur-Gradienten-Programm durchgeführt: 95°C für 3 min; 30 Zyklen mit 95°C für 30 sec, 50°C für 30 sec, und 72°C für 3 min; 72°C für 10 min.; 4°C bis zur Verwendung. Das PCR-Produkt (∼2.2 kB) wurde durch Agarosegel-Elektrophorese (1,2%E-Gel, Invitrogen) und Säulen-Chromatographie (GFX-Kit, Amersham Pharmacia) isoliert und anschließend sequenziert (Sequenzierprimer: Zm-SHC_fw und Zm-S-HC_rev). Die erhaltene Sequenz entspricht der publizierten Sequenz.

Das PCR-Produkt wurde mit den Restriktionsendonukleasen *Nde*I und *Bam*HI verdaut und in entsprechend verdauten *p*DHE19.2-Vektor ligiert. Die Sequenzierung der resultierenden Plasmide ergab die in Seq-ID1 dargestellte Nukleinsäuresequenz.

Das Plasmid *p*DHE-Zm-SHC-1 wurde in den Stamm *E. coli*TG10 *p*Agro4 *p*HSG575 [Takeshita et al., Gene 1987, 61:63-74; Tomoyasu et al., Mol Microbiol2001, 40:397-413] transformiert. Die rekombianten *E. coli* erhalten die Bezeichnung *E. coli* LU**15568**.

### Beispiel 2a: Bereitstellung rekombinanter Homofarnesol-Cyclase aus Z. mobilis (SEQ ID NO 2)

Aus einer entsprechenden 2mL Vorkultur angeimpft, wurde *E. coli*LU**15568** in 20mL LB-Amp/Spec/Cm (100µg/l Ampicillin; 100µg/l Spectinomycin; 20µg/l Chloramphenicol), 0,1 mM IPTG, 0,5g/L Rhamnose in 100mL Erlenmeyerkolben (Schikanen) 16 h bei 37°C angezogen, bei 5000*g/10min zentrifugiert und bei 4°C gelagert. Proteinextrakt wurde hergestellt indem das Zellpellet in 15mL Aufschlusspuffer (0,2M Tris/HCI, 0,5M EDTA, pH 8.0), 375U Benzonase (z.B. Novagen, 25U/µL), 40µL PMSF (100mM, gelöst in *i*-PropOH), 0,8g Saccharose und ca. 0.5mg Lysozym suspendiert wurden. Den Ansatz wurde gemischt und 30min auf Eis inkubiert. Anschließend wurde die Mischung bei -20°C eingefroren.

Nach dem Auftauen des Ansatzes wurden mit Aqua dest. auf ca. 40mL aufgefüllt und nochmals für 30min auf Eis inkubiert.

Anschließend wurden die Zellen 3mal 3min mit Ultraschall aufgeschlossen (HTU-Soni 130, Fa. G. Heinemann, Schwäbisch-Hall, Amplitude 80%, 15" Puls /15" Pause).

Nach dem Aufschluss wurden die Zelltrümmer in 60min bei 4°C und 26900 **g* abzentrifugiert. Der Überstand wurde verworfen und das Pellet in 100mL Solubilisationspuffer (50mM Tris/HCI, 10mM MgCl2x6H2O, 1% Triton X-100, pH 8.0) resuspendiert und ca. 5min gepottert. Anschließend wurde die Suspension für 30min auf Eis gehalten.

Der homogenisierte Extrakt wurde erneut für 1h bei 4°C und 26900 **g* zentrifugiert und das Pellet verworfen. Der Extrakt wurde für die Enzymtests eingesetzt und kann mehrere Wochen ohne Aktivitätsverlust bei -20°C gelagert werden. Der Proteingehalt liegt im Bereich von 1 mg /mL.

### Beispiel 2b: Bereitstellung rekombinanter Homofarnesol-Cyclase aus Bradyrhizobium japonicum (SEQ ID NO 5)

Homofarnesol-Cyclase aus Bradyrhizobium japonicum wurde wie in Beispiel 2a hergestellt.

### Beispiel 3a: Aktivitätsbestimmung der rekombinanten Homofarnesol-Cyclase (SEQ ID NO 2) aus E. coliLU15568

Mit der in Beispiel 2a beschriebenen Proteinpräparation wurde Homofarnesol (**1**, (3*Z*,7*E*)-4,8,12-Trimethyltrideca-3,7,11-trien-1-ol) inkubiert. Im Einzelnen wurden 4mL Proteinpräparation, 0.5mL Na-Citratpuffer (1M Natriumcitrat pH 4.9), 0.5mL Homofarnesol-Lösung (100mM in 0.1M Na-Citratpuffer, pH 6.5 mit 2%(w/w) Taurodesoxycholat) miteinander gemischt und unter Rühren bei 30°C inkubiert. Eine Kontrollreaktion wurde in gleicher Zusammensetzung jedoch bei 60°C inkubiert.

Nach einer 16stündigen Inkubation wurden die Ansätze mit 10mL Hexan/ *n*-Propanol 3:2 extrahiert und die organische Phase zu Trockne eingeengt.

Der Rückstand wurde in 200µL Dichlormethan aufgenommen und für die GC bzw. GC/MS-Analyse eingesetzt.

Mit der in Beispiel 4 dargestellten Analytik konnte ein Umsatz von 41% (»20.5 µmol **2**) festgestellt werden.

Im Vergleich wurde in Umsetzungen von Homofarnesol mit der bekannten Squalen-Hopen-Cyclase aus Alicyclobacillus acidocaldarius 1.2% Umsatz erzielt.

### Beispiel 3b: Aktivitätsbestimmung der rekombinanten Homofarnesol-Cyclase (SEQ ID NO 5) aus E. coli

Die enzymatische Aktivität des in E. coli rekombinant hergestellten Biokatalysators aus Bradyrhizobium japonicum entspricht der Zm-SHC. Nach 3stündiger Inkubation einer 20mM Homofarnesol Lösung mit Zellhomogenat (Proteinmenge: 31 mg) bei 37 °C wurden 26,4% Homofarnesol zum Ambroxan umgesetzt. Unter den gleichen Bedingungen wurden mit rekombinanter Zm-SHC einen Umsatz von 22,4% erzielt.

### Beispiel 4 Analytik

### Umsatz/Quantifizierung

Der Umsatz von Homofarnesol (**1**) zu Ambroxan (**2**) lässt sich mit folgendem GC-System bestimmen:
*Säule*: 10m Optima 1
*Temperaturprofil*:

| | |
|---|---|
| 0': | 100°C |
| | 15°C/min |
| 14.7': | 320°C |
| 34.7' | 320°C |

| | |
|---|---|
| *Injektortemp*: | 320°C |

*RT*: Homofarnesol ca. 12.5'; Ambroxan: ca. 11.4'

Mit authentischem Material wurde eine Eichreihe erstellt, anhand derer die Konzentration unbekannter Proben bestimmt wurde.

### Identifizierung

Die Identifizierung erfolgte mittels Kapillar-GC/MS der positiven Ionen nach Elektronenstoßionisation (EI) und chemischer Ionisation (CI) mit Ammoniak. Geräte: GC (HP 6890) gekoppelt mit zwei MSDs (HP 5973) für EI and CI Ionisation.

**GC BEDINGUNGEN:**

| | | | |
|---|---|---|---|
| Trennsäule: | DB-1 | | |
| Länge: | 30m | | |
| Innendurchmesser: | 250µm | | |
| Filmdicke | 0.25µm | | |
| Trägergas: | He | | |
| Fluss: | 1.2mL/min | | |
| Splitverhältnis: | 1:60 | | |
| Ofentemperatur: | 100°C, 5K/min bis 200°C, 5 min isotherm, 30K/min bis 300°C, 50 min isotherm | | |
| Injektortemperatur: | 250°C | | |
| Transferlinetemperatur: | 300°C | | |
| Dosiermenge: | 0.2µL | | |
| Probenvorbereitung: | Direkte Injektion | | |

**MS BEDINGUNGEN**

| EI: | | CI: | |
|---|---|---|---|
| Scanbereich: | 25- 750 amu | Scanbereich: | 75-800 amu |
| Ionisierungsenergie: | 70 eV | | |

Die vorliegende Anmeldung betrifft demgemäß:
1. Verfahren zur Herstellung von Ambroxan-Derivaten, bevorzugt Ambroxan, der allgemeinen Formel (2) dadurch gekennzeichnet, dass Homofarnesol-Derivate der allgemeinen Formel (1) biokatalytisch zu den entsprechenden Ambroxan-Derivaten mittels eines Polypeptid mit der Aktivität einer Homofarnesol-Ambroxan-Cyclase als Enzym umgesetzt werden. 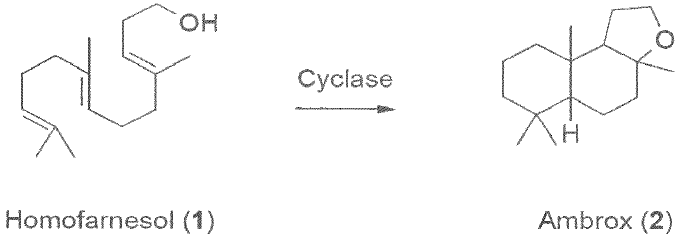
2. Verfahren nach Anspruch 1, dadurch gekennzeichnet, dass das Enzym ein Polypeptid ist, welches kodiert von einem Nukleinsäuremolekül wird, umfassend mindestens ein Nukleinsäuremolekül ausgewählt aus der Gruppe bestehend aus:
   a) Nukleinsäuremolekül, das ein Polypeptid kodiert, umfassend die in SEQ ID NO 2, 5, 6, 7, 8, 9, 10 oder 11;
   b) Nukleinsäuremolekül, das zumindest ein Polynukleotid der Sequenz gezeigt in SEQ ID NO 1 umfasst;
   c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
   d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
   e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Datenbank oder aus einer Genom-Datenbank mittels der Primer gemäß Sequenz Nr. 3 und 4 amplifiziert;
   f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
   g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
   h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
   i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
   j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.
3. Verfahren nach einem der vorhergehenden Ansprüche, dadurch gekennzeichnet, dass das Enzym in einer Form vorliegt, ausgewählt aus der Gruppe bestehend aus:
   a) freies, gegebenenfalls gereinigtes oder teilweise gereinigtes Polypeptid mit der Aktivität einer Homofarnesol-Ambroxan-Cyclase;
   b) immobilisiertes Polypeptid mit der Aktivität einer Homofarnesol-Ambroxan-Cyclase;
   c) aus Zellen isoliertes Polypeptid gemäß a) oder b);
   d) ganze Zelle, gegebenenfalls ruhende oder aufgeschlossene Zellen, enthaltend mindestens ein Polypeptid mit der Aktivität einer Homofarnesol-Ambroxan-Cyclase;
   e) Zelllysat oder Zellhomogenisat der Zellen beschrieben unter d).
4. Verfahren nach Anspruch 3, dadurch gekennzeichnet, dass die Zellen Mikroorganismen sind, bevorzugt transgene Mikroorganismen exprimierend mindestens ein heterologes Nukleinsäuremolekül kodierend für ein Polypeptid mit der Aktivität einer Homofarnesol-Ambroxan-Cyclase.
5. Verfahren nach einem der vorhergehenden Ansprüche, dadurch gekennzeichnet, dass zur Herstellung von Ambroxan als Ausgangsstoff Citral eingesetzt wird, welches in mehreren Schritten zu Homofarnesol umgesetzt wird.
6. Verfahren nach einem der vorhergehenden Ansprüche, dadurch gekennzeichnet, dass die Herstellung von Ambroxan in einphasigen wässrigen Systemen oder in zweiphasigen Systemen erfolgt.
7. Verfahren nach einem der vorhergehenden Ansprüche, dadurch gekennzeichnet, dass die Herstellung von Ambroxan in batch-, fed-batch oder kontinuierlicher Fahrweise erfolgt.
8. Verfahren nach einem der vorhergehenden Ansprüche, wobei die Reaktion von Homofarnesol zu Ambroxan bei einer Temperatur im Bereich von 0 bis 45°C (60°C) und/oder einem pH-Wert im Bereich von 4 bis 8 erfolgt.
9. Verfahren nach einem der vorhergehenden Ansprüche, dadurch gekennzeichnet daß die Homofarnesol-Ambroxan-Cyclase aus einem Mikroorganismus, welcher die Homofarnesol-Ambroxan-Cyclase überexprimiert, ausgewählt aus der Gruppe Bakterien der Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.
10. Verfahren nach einem der vorhergehenden Ansprüche, dadurch gekennzeichnet daß die Homofarnesol-Ambroxan-Cyclase aus einem Mikroorganismus ausgewählt aus der Gruppe bestehend aus Methylococcus capsalatus, Rhodopseudomonas palustris, Frankia spec., Streptomyces coelicolor, Rhodopseudomonas palent, Frankia alni, Bacillus anthracis, Burkholderia ambifaria, insbesondere Zymomonas mobilis und Bradyrhizobium japonicum isoliert wurde.
11. Verfahren nach einem der vorhergehenden Ansprüche, dadurch gekennzeichnet daß die Homofarnesol-Ambroxan-Cyclase aus transgenen Bakterien der Spezies Escherichia coli, Pseudomonas putida, Burkholderia glumae, Streptomyces lividans, Streptomyces coelicolor und Methylococcus capsalatus, Rhodopseudomonas palustris, Frankia spec., Rhodopseudomonas palent, Frankia alni, Bacillus anthracis, Burkholderia ambifaria, insbesondere Zymomonas mobilis und Bradyrhizobium japonicum isoliert wurde, welche die Homofarnesol-Ambroxan-Cyclase überexprimieren.
12. Verwendung eines Polypeptids mit der Aktivität einer Homofarnesol-Ambroxan-Cyclase zur biokatalytischen Umsetzung von Homofarnesol zu Ambroxan.
13. Verwendung nach Anspruch 12, dadurch gekennzeichnet, dass das Polypeptids kodiert wird von einem Nukleinsäuremolekül umfassend mindestens ein Nukleinsäuremolekül ausgewählt aus der Gruppe bestehend aus:
   a) Nukleinsäuremolekül, das ein Polypeptid kodiert, umfassend die in SEQ ID NO 2, 5, 6, 7, 8, 9, 10 oder 11;
   b) Nukleinsäuremolekül, das zumindest ein Polynukleotid der Sequenz gezeigt in SEQ ID NO 1 umfasst;
   c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
   d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
   e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Bank oder aus genomischer DNA mittels der Primer gemäß Sequenz Nr. 3 und 4 amplifiziert bzw. durch *de novo-*Gensynthese herstellt.;
   f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
   g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
   h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
   i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
   j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.
14. Genkonstrukt oder Vektor umfassend ein Nukleinsäuremolekül kodierend für ein Polypeptid mit der Aktivität einer Homofarnesol-Ambroxan-Cyclase, bevorzugt ein Nukleinsäuremolekül umfassend mindestens ein Nukleinsäuremolekül ausgewählt aus der Gruppe bestehend aus:
   a) Nukleinsäuremolekül, das ein Polypeptid kodiert, umfassend die in SEQ ID NO 2, 5, 6, 7, 8, 9, 10 oder 11;
   b) Nukleinsäuremolekül, das zumindest ein Polynukleotid der Sequenz gezeigt in SEQ ID NO 1 umfasst;
   c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
   d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
   e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Bank oder aus genomischer DNA mittels der Primer gemäß Sequenz Nr. 3 und 4 amplifiziert bzw. durch *de novo-*Gensynthese herstellt.;
   f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
   g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
   h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
   i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
   j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen .
15. Wirtszelle enthaltend ein Genkonstrukt oder einen Vektor gemäß Anspruch 14.

## Patentansprüche

1. Verfahren zur Herstellung von Ambroxan-Derivaten, bevorzugt Ambroxan, der allgemeinen Formel (2) **dadurch gekennzeichnet, dass** Homofarnesol-Derivate der allgemeinen Formel (1) biokatalytisch zu den entsprechenden Ambroxan-Derivaten mittels eines Polypeptid mit der Aktivität einer Homofarnesol-Ambroxan-Cyclase als Enzym umgesetzt werden 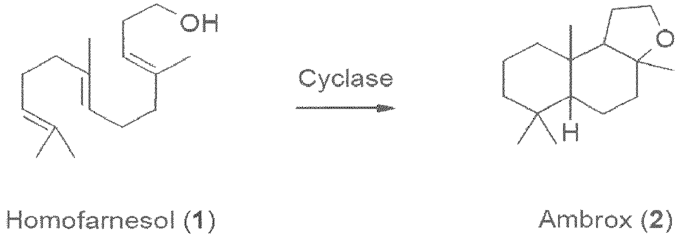 **dadurch gekennzeichnet, dass** das Enzym ein Polypeptid ist, welches kodiert von einem Nukleinsäuremolekül wird, umfassend mindestens ein Nukleinsäuremolekül ausgewählt aus der Gruppe bestehend aus:
a) Nukleinsäuremolekül, das ein Polypeptid kodiert, umfassend die in SEQ ID NO 6, 7, 8, 9 oder 11;
b) Nukleinsäuremolekül, das ein Polypeptid kodiert, dessen Sequenz eine Identität von mindestens 46% zu den Sequenzen SEQ ID NO 6, 7, 8, 9 oder 11 aufweist;
c) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
d) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Datenbank oder aus einer Genom-Datenbank mittels der Primer gemäß Sequenz Nr. 3 und 4 amplifiziert;
e) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
f) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
g) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
h) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
i) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

2. Verfahren nach Anspruch 1, **dadurch gekennzeichnet, dass** das Enzym in einer Form vorliegt, ausgewählt aus der Gruppe bestehend aus:
a) freies, gegebenenfalls gereinigtes oder teilweise gereinigtes Polypeptid mit der Aktivität einer Homofarnesol-Ambroxan-Cyclase;
b) immobilisiertes Polypeptid mit der Aktivität einer Homofarnesol-Ambroxan-Cyclase;
c) aus Zellen isoliertes Polypeptid gemäß a) oder b);
d) ganze Zelle, gegebenenfalls ruhende oder aufgeschlossene Zellen, enthaltend mindestens ein Polypeptid mit der Aktivität einer Homofarnesol-Ambroxan-Cyclase;
e) Zelllysat oder Zellhomogenisat der Zellen beschrieben unter d).

3. Verfahren nach Anspruch 2, **dadurch gekennzeichnet, dass** die Zellen Mikroorganismen sind, bevorzugt transgene Mikroorganismen exprimierend mindestens ein heterologes Nukleinsäuremolekül kodierend für ein Polypeptid mit der Aktivität einer Homofarnesol-Ambroxan-Cyclase.

4. Verfahren nach einem der vorhergehenden Ansprüche, **dadurch gekennzeichnet, dass** zur Herstellung von Ambroxan als Ausgangsstoff Citral eingesetzt wird, welches in mehreren Schritten zu Homofarnesol umgesetzt wird.

5. Verfahren nach einem der vorhergehenden Ansprüche, **dadurch gekennzeichnet, dass** die Herstellung von Ambroxan in einphasigen wässrigen Systemen oder in zweiphasigen Systemen erfolgt.

6. Verfahren nach einem der vorhergehenden Ansprüche, **dadurch gekennzeichnet, dass** die Herstellung von Ambroxan in batch-, fed-batch oder kontinuierlicher Fahrweise erfolgt.

7. Verfahren nach einem der vorhergehenden Ansprüche, wobei die Reaktion von Homofarnesol zu Ambroxan bei einer Temperatur im Bereich von 0 bis 45°C (60°C) und/oder einem pH-Wert im Bereich von 4 bis 8 erfolgt.

8. Verfahren nach einem der vorhergehenden Ansprüche, **dadurch gekennzeichnet daß** die Homofarnesol-Ambroxan-Cyclase aus einem Mikroorganismus, welcher die Homofarnesol-Ambroxan-Cyclase überexprimiert, ausgewählt aus der Gruppe Bakterien der Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.

9. Verfahren nach einem der vorhergehenden Ansprüche, **dadurch gekennzeichnet daß** die Homofarnesol-Ambroxan-Cyclase aus einem Mikroorganismus ausgewählt aus der Gruppe bestehend aus Methylococcus capsalatus, Rhodopseudomonas palustris, Frankia spec., Streptomyces coelicolor, Rhodopseudomonas palent, Frankia alni, Bacillus anthracis, Burkholderia ambifaria, insbesondere Zymomonas mobilis und Bradyrhizobium japonicum isoliert wurde.

10. Verfahren nach einem der vorhergehenden Ansprüche, **dadurch gekennzeichnet daß** die Homofarnesol-Ambroxan-Cyclase aus transgenen Bakterien der Spezies Escherichia coli, Pseudomonas putida, Burkholderia glumae, Streptomyces lividans, Streptomyces coelicolor und Methylococcus capsalatus, Rhodopseudomonas palustris, Frankia spec., Rhodopseudomonas palent, Frankia alni, Bacillus anthracis, Burkholderia ambifaria, insbesondere Zymomonas mobilis und Bradyrhizobium japonicum isoliert wurde, welche die Homofarnesol-Ambroxan-Cyclase überexprimieren.

11. Verwendung eines Polypeptids mit der Aktivität einer Homofarnesol-Ambroxan-Cyclase zur biokatalytischen Umsetzung von Homofarnesol zu Ambroxan.

12. Verwendung nach Anspruch 11, **dadurch gekennzeichnet, dass** das Polypeptids kodiert wird von einem Nukleinsäuremolekül umfassend mindestens ein Nukleinsäuremolekül ausgewählt aus der Gruppe bestehend aus:
a) Nukleinsäuremolekül, das ein Polypeptid kodiert, umfassend die in SEQ ID NO 6, 7, 8, 9 oder 11;
b) Nukleinsäuremolekül, das ein Polypeptid kodiert, dessen Sequenz eine Identität von mindestens 46% zu den Sequenzen SEQ ID NO 6, 7, 8, 9 oder 11 aufweist;
c) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
d) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Bank oder aus genomischer DNA mittels der Primer gemäß Sequenz Nr. 3 und 4 amplifiziert bzw. durch *de novo*-Gensynthese herstellt.;
e) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
f) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
g) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
h) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
i) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

13. Genkonstrukt oder Vektor umfassend ein Nukleinsäuremolekül kodierend für ein Polypeptid mit der Aktivität einer Homofarnesol-Ambroxan-Cyclase, bevorzugt ein Nukleinsäuremolekül umfassend mindestens ein Nukleinsäuremolekül ausgewählt aus der Gruppe bestehend aus:
a) Nukleinsäuremolekül, das ein Polypeptid kodiert, umfassend die in SEQ ID NO 2, 5, 6, 7, 8, 9, 10 oder 11;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Bank oder aus genomischer DNA mittels der Primer gemäß Sequenz Nr. 3 und 4 amplifiziert bzw. durch *de novo*-Gensynthese herstellt.;
f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

14. Wirtszelle enthaltend ein Genkonstrukt oder einen Vektor gemäß Anspruch 13.
